Europäisches Patentamt

European Patent Office

Office européen des brevets

(19)

(11) Publication number : **0 494 840 A1**

(12) # EUROPEAN PATENT APPLICATION

(21) Application number : **92500003.6**

(22) Date of filing : **10.01.92**

(51) Int. Cl.$^5$ : **A61B 1/24, A61B 13/00**

(30) Priority : **11.01.91 ES 9100088 U**

(43) Date of publication of application :
**15.07.92 Bulletin 92/29**

(84) Designated Contracting States :
**BE CH DE DK FR GB IT LI NL PT**

(71) Applicant : **Cobo de la Cruz, José
Alonso Cano 44
E-28003 Madrid (ES)**

(72) Inventor : **Cobo de la Cruz, José
Alonso Cano 44
E-28003 Madrid (ES)**

(74) Representative : **Garcia Cabrerizo, Francisco
OFICINA GARCIA CABRERIZO S.L. Vitruvio 23
E-28006 Madrid (ES)**

(54) Disposable sterile lingual anatomical depressor for the exploration of the isthmus of the fauces and the pharynx.

(57) A disposable sterile anatomical lingual depressor for the exploration of the isthmus of the fauces and the pharynx, which is made up of a thin transparent plastic body and which may be differentiated into three distinct parts, one acting as a depressor plate (1), another acting as a handle (3) and the third acting as a middle section (2) and separating the former two ; with the special feature that the part constituting the depressor plate has a slighty concave upper face along all its length, whereas its lower face is upwardly convex, along the sagittal plane, having along its longitudinal axis a central protuberance (7) forming a rounded elevation intended to match the shape of and press onto the tongue ; the handle has on its upper and lower sides a recess providing support (4) to a matching the shape of the user's index finger and thumb ; the middle section or separator between the depressor plate and the handle is oblique and forms an obtuse angle with the proximal end of the plate, causing same to be on a different plane to that of the handle.

Fig. 2

EP 0 494 840 A1

The present invention refers to an instrument known as a disposable anatomical tongue depressor for the exploration of the isthmus of the fauces and the pharynx, with the aim of achieving the maximum effectiveness, efficacy, safety and ease of handling in exploration, as well as full acceptation by the patient, regardless of age (baby, child, adult).

For the examination of the pharynx and the isthmus of the fauces, several instruments, suited to a greater or lesser degree to the purpose, are commonly used. The typical operation consists in telling the patient to relax and to breathe in through the mouth without putting out their tongue; using the right hand, the tongue depressor is introduced into the patient's mouth, avoiding touching the tongue or just lightly rubbing against it to reassure the patient; then the tongue is depressed exerting a light constant pressure, taking special care to avoid it being rejected out of the mouth and making sure that the operation does not take too long, since this might prompt a reflex of nausea; likewise, if the tongue proves too unmanageable, the physician must act patiently, repeating the explorations frequently and for short period of time.

The physician will aid his exploration holding in this left hand a lamp or pocket torch or any source of light, including a frontal mirror to provide an excellent vision. It is highly recommendable to use the front mirror with the source of light directed towards the mouth cavity.

Currently, the most commonly used tongue depressor is the wooden depressor stick, used only once, consisting of a rigid horizontal flat rectangular strip of wood, measuring 14 cm long by 16 mm wide and 2 mm thick. There exists one model only. The sides end in corners (they are not rounded) and the two corners of each end are canted off.

Metal tongue depressors are no longer used due to the need for prior sterilisation in order to treat the next patient. This type of depressor consists of a depressor plate and a handle joined at an angle.

The depressors currently in use present a number of problems and disadvantages which detract from the efficiency of the exploration, notably the following ones:

– Depressor sticks, being made of wood, evidently leave a taste of wood since the tongue is the single and specific location of the sense of taste.

– Such sticks are not sterile, that is to say, they are not presented as a single and independent sterilised unit in sterile packaging. Wood is a material which is virtually impossible to sterilise.

– They are available in only one size, there are no baby or child sizes.

– The practitioner's vision is hindered by having to interpose his right hand (the wooden stick is horizontal along its full length), holding the outer end of the stick, in the visual field to be explored.

– No tongue depressor adapts itself to the shape and anatomical constitution of the tongue; therefore, anatomical factors are not taken into account in order to ensure greater effectiveness and ease of handling in the exploration operation.

– The material used in these depressors, being opaque, makes it impossible to view the contact area and the length of depressor resting on the tongue, with the risk of introducing the depressor too far in giving rise to reflex nausea and hindering manoeuvrability.

– Wooden depressor sticks are unsafe, since they may break along weak points in the grain of the wood, or they may not be smooth enough, and furthermore, all edges are pointed.

– Their shape is not adapted to the practitioner's fingers, and therefore they do not ensure maximum ease of handling.

– Being made of an opaque material, they hinder or do not improve the transmission of light.

– They are exposed to bacterial contamination (doctors' surgeries, hospitals, manipulators) and act as a vehicle of contagion.

– Given that they do not have an anatomical design, their use requires a stronger pressure on the tongue, causing discomfort to the patient.

The proposed invention aims to solve the foregoing problems, by means of the design of an instrument known as a disposable and sterile anatomical lingual depressor, for the exploration of the isthmus of the fauces and the pharynx, which meets the requirements for a suitable exploration:

– It ensures a wide field of vision, thus allowing maximum effectiveness of exploration.

– The instrument under reference is disposable, sterile, easy to handle, practical and safe; furthermore, it follows and takes advantage of the morphology and anatomical features of the tongue.

– It does not produce rejection, unnecessary discomfort, nausea, unpleasant taste or accidents, and it is fully suited to the dimensions of the mouth cavity (babies, children and adults).

Based on the above aims, its design represents numerous advantages for doctors and patients.

More specifically, for the purposes of a proper description, the object of the present invention may, in a figurative sense, be divided according to its distinct characteristics into three parts:

– Depressor plate

– Angle section joining depressor plate and handle

– Handle.

Any of the three above parts is a continuation of the two others, there being no separation or joints between them. The constituent material is exclusively transparent plastic, sufficiently resistant to avoid breakages are bending when performing lingual depression operations.

As regards the depressor plate, i.e. the part of the depressor which enters into contact with the tongue, said part has three very particular and distinct features, adapted to the anatomical features and morphology of the tongue:

- It has along the sagittal axis an upwards convexity in the distal third of the plate.
- Frontally, the lower face is upwardly convex along the whole length of the plate.
- Also fontally, the upper face has a slight upwards concavity along the whole length of the plate.

The dimensions of said depressor plate are designed according to the dimensions of the mouth cavity of babies, children (3-12 years) and adults.

In detail, each of the parts of the depressor plate exhibits the following features.

Upper face: slight concavity in frontal sense (2 mm at the centre) said concavity extends along the full length of the plate. This concavity improves the visual field and at the same time, its minimum thickness ensures the minimum interference with the visual field. The lateral edges and the distal end are rounded or bevelled (there are no sharp edges).

Lower face: It is upwardly convex, along the sagittal axis, having along its middle a blunt protuberance or elevation, designed to adapt to the shape of the sulcus medianus linguae (through or depression along the middle of the tongue) and to press on the lingual septum, in order to exert a stronger physiological pressure on the required area and flatten the tongue, distancing it from the palate, leaving the greatest possible clear space for viewing.

The aim of this design is to exert the maximum pressure upon the sulcus medianus linguae, the lingual septum and the hyoglossal and genioglossal muscles, as well as the rest of the tongue muscular mass.

Distal end: The tip of the depressor plate is slightly curved. This design corresponds to the V-shaped form of the sulcus terminalis, where there are located the calceiform buds, arranged in a V shape to take part in tasting. Given that this area is bound to produce nauseous reactions, the curved finish of the distal end is appropriate to avoid resting on the V-shape of the calceiform taste buds.

Proximal end: It extends continuously into the angle section.

Longitudinal design: The foregoing features are complemented by the slight concavity of the distal third of the plate, with the rest of the plate's length horizontal.

As regards the angle section joining the plate and the handle, it is worth noting that it breaks the horizontality of the plate, forming a 140° angle with the proximal end of same. The inclination of the angle section improves manoeuvrability, avoiding disturbance of the lower lip or the dental arch. The angle section has the main function of preventing the hand holding the instrument from interfering with the field of vision. The angle section removes the fingers from the visual field of exploration.

The handle constitutes the means of holding the instrument and has a rectangular shape, following as a continuation of the angle section. It has in its upper face a recess providing support for the index finger, and a similar recess in the lower face for the thumb. Said recesses allow a suitable grip on the instrument and facilitate its handling.

Major advantages include the following:

- It is tasteless, therefore leaving no aftertaste at the end of the exploration (taste of wood, disinfectant, etc.).
- It helps to achieve the maximum field of exploration, by adapting to the morphological and anatomical features of the tongue.
- It does not interfere with the exploration visual field when held by the hand performing the exploration.
- The handle with finger support recesses provides easy handling.- Being made of transparent material, it allows observation and identification of the tongue area in contact, which avoids causing nausea and vomiting.
- The size of the depressor is adapted to the patient's mouth cavity, depending on whether it is a baby, a child or an adult.
- It is safe, due to its extreme resistance to breakages.

All edges are rounded and bending is nonexistent.

- Its presentation as a single disposable sterile unit avoids contamination and possible contagion.

In order to ensure the perfect understanding of the invention, a description is set out below based on a sheet of drawings attached to the present description, showing the following:

Figure 1 shows a top view of the anatomical depressor of the invention.

Figure 2 shows a longitudinal section of the same instrument.

Figure 3 shows a bottom view, featuring the lower face.

Figure 4 shows a transverse section of the depressor plate.

Figure 5 shows a transverse section of the handle.

In these figures, the numbers correspond to the following parts and elements:

1.- Depressor plate. Upper face.

2.- Angle section joining the depressor plate and the handle.

3.- Handle.

4.- Recess or hollow providing support for the index finger.

5.- Curvature of the distal end of the depressor plate.

6.- Depressor plate. Lower face.

7.- Protuberance on lower face for adaptation to lingual anatomy.

8.- Recess or hollow providing support for the thumb.

As can be seen in the figures, the instrument known as a disposable sterile anatomical depressor is made up of: depressor plate (1), angle section (2) and handle (3).

The depressor plate (1) shall measure preferably 9 cm long and 3.3 cm wide at the distal end and 2.2 cm at the proximal end. Its thickness is of 4 mm parallel to its lateral sides. These dimensions correspond to the adult version.

The children's version should preferably have the following measurements: 6 cm long, 2.2 cm wide at the proximal end and 2.6 cm at the distal end, with a thickness of 4 mm.

The baby version should preferably have the following measurements: 5 cm long, 1.5 cm at the proximal end and 1.8 cm at the distal end, with a tickness of 4 mm.

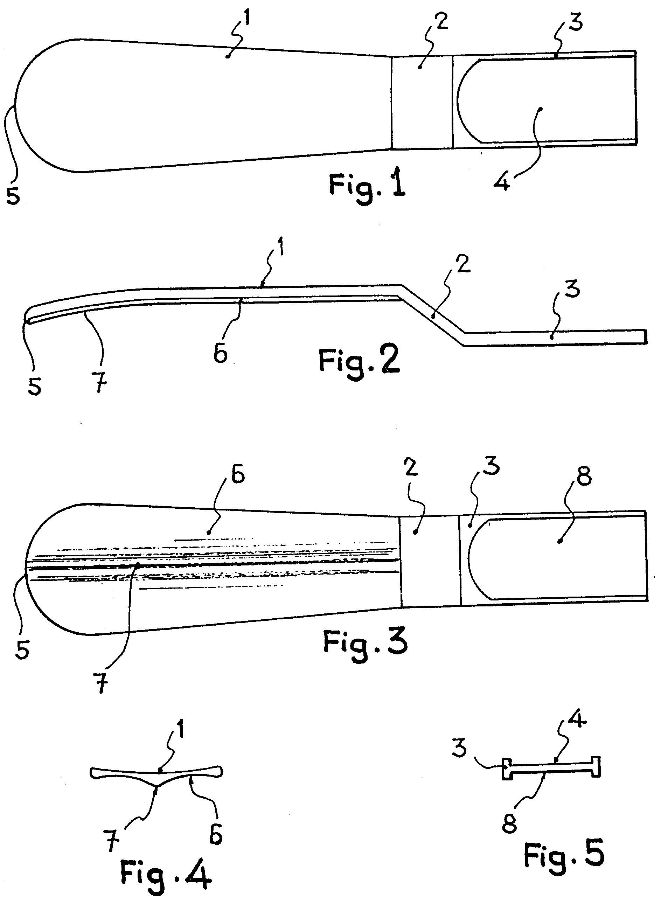

The depressor plate features on its upper face a concavity (2 mm in the middle) as shown in figure 4.

The lower face of said depressor plate features in the centre longitudinally a rounded protuberance (7) measuring 4 mm approximately. The rest of said face is upwardly convex (fig. 4).

The distal third, lengthwise, of the plate features on the sagittal plane a slight upwards convexity, ending in a curved tip (5) (fig. 2).

The angle section (2) is at an angle of 140 to the horizontal plane of the handle (3) (fig. 2) and measures 1.5 in length, 2.2 in width and 4 mm thick.

The handle (3) shall measure preferably 4.5 cm long, 2.2 cm wide 4 mm thick. It has a hollow or recess (4) on its upper face and another (8) on its lower face, approximately 1.2 mm deep (figs. 1, 2 and 5).

The dimensions of the handle and the angle section (2) are the same for all versions (baby, infant and adult).

This instrument is easy to use: the physician performing the exploration takes the instrument with his/her hand, holding the handle, designed for easier operation, between index finger and thumb, the fingers thus remain outside the field of view thanks to the angle section. The practitioner will then introduce the depressor plate into the mouth cavity ensuring that the lower face matches the lingual features, aided in this respect by the transparent nature of the instrument, and presses the plate down onto the tongue, thus providing a perfect view of the exploration field, namely the isthmus of the fauces and the pharynx.

**Claims**

1.- A disposable sterile anatomical lingual depressor for the exploration of the isthmus of the fauces and the pharynx, characterized by being made up of a thin transparent plastic body, which may be differentiated into three distinct parts, one acting as a depressor plate, another acting as a handle and the third acting as a middle section and separating the former two; with the special feature that the part constituting the depressor plate has a slightly concave upper face along all its length, whereas its lower face is upwardly convex, along the sagittal plane, having along its longitudinal axis a central protuberance forming a rounded elevation intended to match the shape of and press onto the tongue; the handle has on its upper and lower sides a recess providing support to and matching the shape of the user's index finger and thumb; the middle section or separator between the depressor plate and the handle is oblique and forms an obtuse angle with the proximal end of the plate, causing same to be on a different plane to that of the handle.

2.- A disposable sterile anatomical lingual depressor for the exploration of the isthmus of the fauces and the pharynx, according to claim 1, characterized in that the depressor plate has bevelled edges, forming a rounded perimeter; said plate being light and becoming wider towards the distal end, which end finishes in an arched shape, with the particular feature that on the sagittal plane of said plate, the distal third features a slight upwards convexity along its longitudinal axis.

Fig. 1

Fig. 2

Fig. 3

Fig. 4

Fig. 5

**European Patent Office**

## EUROPEAN SEARCH REPORT

Application Number

EP 92 50 0003

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-2 723 661 (G.P.HULL) | 1 | A61B1/24 |
| A | * column 2, line 7 - column 3, line 22; figures 1-7 * | 2 | A61B13/00 |
| | --- | | |
| A | US-A-2 653 597 (W.T.CANAN) | 1,2 | |
| | * column 2, line 16 - line 45; figures 1-3 * | | |
| | --- | | |
| A | US-A-3 863 627 (P.BOUFFARD) | 1,2 | |
| | * column 1, line 61 - column 2, line 48; figures 1-3 * | | |
| | ----- | | |
| | | | **TECHNICAL FIELDS SEARCHED (Int. Cl.5 )** |
| | | | A61B |

The present search report has been drawn up for all claims

| Place of search | Date of completion of the search | Examiner |
|---|---|---|
| BERLIN | 06 APRIL 1992 | WEIHS J. |

EPO FORM 1503 03.82 (P0401)